# FASCICULE DE BREVET EUROPEEN

(11) **EP 1 292 333 B1**
(45) Date de publication et mention de la délivrance du brevet: **07.02.2007**
(21) Numéro de dépôt: 01945376.0
(22) Date de dépôt: 07.06.2001
(51) Int. Cl.: A61K 39/39, A61P 31/04, A61P 33/00, A61P 37/04

(54) **COMPOSITION ADJUVANTE COMPRENANT LA PROTEINE FHA OU UN FRAGMENT DE LA PROTEINE FHA SOUS FORME LIBRE**
ADJUVANSZUSAMMENSETZUNG ENTHALTEND FHA ODER EIN FHA-FRAGMENT IN FREIER FORM
ADJUVANT COMPOSITION COMPRISING FHA PROTEIN OR FRAGMENT OF FHA PROTEIN IN FREE FORM

(30) Priorité: 07.06.2000 FR 0007302
(43) Date de publication de la demande: 19.03.2003
(73) Titulaire: INSTITUT PASTEUR DE LILLE, 59019 Lille Cédex (FR); INSTITUT NATIONAL DE LA SANTE ET DE LA RECHERCHE MEDICALE (INSERM), 75654 Paris Cédex 13 (FR)
(72) Inventeur: CAPRON, André, F-59133 Phalempin (FR); LOCHT, Camille, B-1180 Bruxelles (BE); MENOZZI, Franco, B-7022 Mons-Hyon (BE); POULAIN-GODEFROY, Odile, F-59130 Lambersart (FR); RIVEAU, Gilles, F-59133 Phalempin (FR)
(74) Mandataire: Desaix, Anne
(86) Numéro de dépôt international: PCT/FR2001/001769
(87) Numéro de publication internationale: WO 2001/093906

(56) Documents cités:
- EP-A- 0 267 998
- EP-A- 0 471 177
- WO-A-96/31535
- US-A- 5 895 655
- CAHILL E S ET AL: "Immune responses and protection against Bordetella pertussis infection after intranasal immunization of mice with filamentous haemagglutinin in solution or incorporated in biodegradable microparticles" VACCINE,GB,BUTTERWORTH SCIENTIFIC. GUILDFORD, vol. 13, no. 5, 1995, pages 455-462, XP004057720 ISSN: 0264-410X
- SHAHIN R D ET AL: "MUCOSAL IMMUNIZATION WITH FILAMENTOUS HEMAGGLUTININ PROTECTS AGAINST BORDETELLA-PERTUSSIS RESPIRATORY INFECTION" INFECTION AND IMMUNITY, vol. 60, no. 4, 1992, pages 1482-1488, XP002160230 ISSN: 0019-9567
- POULAIN-GODEFROY ODILE ET AL: "Bordetella pertussis filamentous hemagglutinin enhances the immunogenicity of liposome-delivered antigen administered intranasally." INFECTION AND IMMUNITY, vol. 66, no. 4, avril 1998 (1998-04), pages 1764-1767, XP002160231 ISSN: 0019-9567 cité dans la demande

## Description

La présente invention se situe dans le domaine des adjuvants pour la vaccination et plus particulièrement des adjuvants permettant aussi bien la stimulation de l'immunité par voie mucosale que systémique.

La présente invention concerne toute utilisation de la protéine FHA ou une partie de la protéine FHA, sous forme libre, pour la préparation d'une composition adjuvante de la réponse immunitaire à usage humain ou animal.

Elle est également relative à toute composition adjuvante de la réponse immunitaire comprenant la protéine FHA ou une partie de la protéine FHA, sous forme libre.

L'hémagglutinine filamenteuse (FHA) de *Bordetella pertussis* est une adhésine majeure produite et sécrétée par la bactérie. Le gène de structure de la FHA code pour une protéine de 367 KDa, et la forme mature est constituée des 60 % N-terminaux de ce précurseur (références 1 à 5).

Les fonctions des parties N-terminale et C-terminale du précurseur (fhaB) et celle de la protéine mature sécrétée (FHA) sont décrites dans Geneviève Renauld - Mongénie et al (6). Il apparaît que le domaine N-terminal de fhaB joue un rôle essentiel dans la sécrétion de la protéine mature puisqu'une délétion en phase de cette région semblé totalement inhiber ladite sécrétion.

A l'état mature, la protéine FHA a un poids moléculaire de 220 kDa et présente au moins trois sites de liaison caractérisés (1) :
- un site de liaison aux carbohydrates qui permet sa fixation aux cellules épithéliales ciliées,
- un site de liaison à l'héparine et aux carbohydrates sulfatés impliqués dans l'attachement aux cellules épithéliales non ciliées et à la matrice extra-cellulaire ;
- une séquence RGD qui permet l'attachement aux macrophages par l'intermédiaire de leurs intégrines.

La FHA est une adhésine produite par différentes souches de *Bordetella.* On peut citer notamment les adhésines filamenteuses produites par *Bordetella bronchiseptica* et *Bordetella parapertussis.* Ces différentes FHA *de Bordetella* présentent un pourcentage d'homologie d'au moins 60 % avec la séquence de la FHA de *Bordetella pertussis.* En outre, la région N-terminale de la FHA présente de fortes similarités de séquences avec les domaines N-terminaux de *S. marcescens* ou de *P. mirabilis* (4).

Dans l'ensemble de ce texte, toute hémagglutinine filamenteuse ayant au moins 60 % d'homologie des séquences avec la FHA de *B. pertussis,* ou tout polypeptide ayant une homologie de séquences d'au moins 60 % et de préférence 70 % avec la FHA de *B. pertussis* doit être considéré comme un équivalent fonctionnel de la FHA de *B. pertussis.*

Les propriétés d'adhérence de la FHA à différents types cellulaires, comme les cellules épithéliales ciliées et non ciliées ou encore les monocytes/macrophages ont été mises à profit dans l'état de la technique afin de cibler la présentation d'un antigène d'intérêt vers ces cellules qui peuvent être responsables de la présentation de l'antigène au système immunitaire, le cas échéant après maturation.

Plus particulièrement, les propriétés des hémagglutinines filamenteuses de *Bordetella* de se fixer aux cellules épithéliales non ciliées et à la matrice extra-cellulaire permettent d'envisager un ciblage vers les muqueuses.

L'immunisation par voie muqueuse offre des avantages certains par rapport à une immunisation par voie parentérique. D'une part, une immunisation par voie muqueuse permet une stimulation simultanée de l'immunité muqueuse et de l'immunité systémique, ce qui n'est pas le cas de la voie systémique. D'autre part, la voie muqueuse est une voie d'administration qui provoque peu d'effets secondaires. Enfin, le développement de vaccins adaptés aux exigences des pays en voie de développement passera certainement par une administration par voie muqueuse. Cependant, la plupart des antigènes sont faiblement immunogènes quand ils sont administrés par voie muqueuse, probablement parce que leur interaction avec le système immunitaire muqueux est faible. Différentes stratégies ont été développées pour augmenter l'immunagénicité des antigènes administrés par cette voie, d'une part en favorisant leur accès à la muqueuse, d'autre part en utilisant des processus capables d'augmenter la réponse obtenue.

L'accès à la muqueuse est favorisé par des antigènes de type particulaire dont l'interaction avec les tissus lymphoïdes est facilitée. Les formes particulaires les plus classiquement développées sont de plusieurs types: liposomes (7), microsphères (8), ISCOMs (9). Ce sont tous des structures globulaires synthétiques qui encapsulent l'antigène à administrer et dont la composition et leur mode de fabrication diffèrent selon leur nature (lipides pour les liposomes, polymères organiques pour les microsphères, mélange de lipides et de Quil A pour les ISCOMs). Une autre approche consiste à utiliser des microorganismes recombinants exprimant l'antigène d'intérêt. Ces microorganismes peuvent être vivants ou inactivés, et ils sont utilisés comme vecteur amenant l'antigène au niveau du système immunitaire sous-jacent des muqueuses. Ainsi, les Samonelles ont été utilisées comme vecteur vivant pour initier une immunisation par voie orale. Un vecteur vivant adapté à l'immunisation par le tractus respiratoire a été réalisé grâce au développement de souches recombinantes de *Bordetella pertussis.* Ces stratégies utilisant des vaccins vivants présentent en outre l'intérêt de permettre une exposition prolongée de l'antigène aux muqueuses de l'hôte lors de la colonisation et ne nécessitent donc pas l'emploi d'adjuvants.

Le développement d'adjuvants efficaces par voie muqueuse s'est fait dans un premier temps en testant les adjuvants qui avaient prouvé leur efficacité par voie parentérique, comme les composés de la famille de muramyl-peptides. Les adjuvants muqueux les plus efficaces à ce jour restent les toxines bactériennes telles que la toxine cholérique, la toxine de *E. coli,* et pour un moindre développement la toxine pertussique. Un des problèmes majeurs de l'utilisation de ces toxines comme adjuvants muqueux est leur toxicité et de nombreux auteurs ont cherché à construire des mutants de ces toxines dépourvus d'activité toxique mais ayant conservé une activité adjuvante. II existe cependant encore un réel besoin d'adjuvants muqueux efficaces et sans effets secondaires.

Les inventeurs ont cherché dans une première approche à augmenter l'adhérence des liposomes aux muqueuses, et par cela même de limiter les quantités d'antigène nécessaires à une immunisation efficace, en couplant l'antigène des préparations liposomales à la FHA. L'addition de FHA à des liposomes contenant un antigène tel que la Sm 28 GST de *Schistosoma mansoni* permet d'augmenter la réponse immune obtenue par voie nasale contre cet antigène. L'explication immédiate de ce résultat était, compte tenu des propriétés d'adhérence de la FHA, que cette dernière permet une meilleure adhérence des liposomes aux surfaces mucosales et permet ainsi de faciliter leur accès aux cellules du système immunitaire. C'est ce concept qui a été validé dans le brevet WO 98/16553; où Mielcarek et al décrivent des constructions hybrides entre tout ou partie de la FHA et tout ou partie d'une protéine antigénique hétérologue vis-à-vis de la FHA. Les résultats expérimentaux qui y sont décrits conduisent à l'hypothèse que la réponse immune obtenue à l'égard de la protéine hétérologue résulte du fait que la FHA permet une meilleure adhésion des liposomes aux surfaces muqueuses C'est sur ces caractéristiques fonctionnelles que la demande de brevet supra décrit effectivement l'utilisation de la FHA comme molécule de ciblage en surface des différents vecteurs vaccinaux (liposomes, microsphères, etc.) pour augmenter la réponse immune induite par l'utilisation de liposomes.

Les protéines hybrides entre la FHA et la glutathion S-transférase de *Schistosoma mansoni* (Sm 28 GST) permettaient l'obtention d'une réponse immune vis-à-vis de la Sm 28 GST et protectrice vis-à-vis de l'infection par *Schistosoma mansoni* (12).

Les auteurs ont précisé que l'ajout de FHA à des liposomes déjà fabriqués avec l'antigène d'intérêt seul a donné des résultats satisfaisants ; autrement dit, la FHA qui possède des séquences hydrophobes qui lui permettent de s'insérer facilement dans les liposomes et des séquences hydrophiles, présentant des propriétés d'adhésine de la FHA permet à la fois le ciblage des cellules immuno-compétentes, et des différents sites du tractus respiratoire.

Les auteurs en déduisent qu'il est possible et suggéré d'utiliser d'autres vecteurs synthétiques du même type, par exemple des vecteurs portant, outre la FHA, plusieurs antigènes différents pour la réalisation de formulations vaccinales multi-antigéniques.

WO 96/31535 décrit la purification de la FHA par un procédé de fractionnement par chromatographie sur gels d'affinité. La FHA ainsi purifiée est utilisée pour la production de vaccins. C'est donc les propriétés d'antigène ou d'immunogène en tant que telles de la FHA qui sont ici évoquées.

EP 0 267 996 porte également sur la FHA en tant qu'antigène vaccinant, et donc apte à solliciter une réponse spécifique et non polyvalente.

Cahill et al. porte également sur une réponse spécifique à la FHA en tant qu'antigène et donc la production d'IgG spécifiques de la FHA. Ceci est bien précisé, y compris dans le résumé où il n'est question que des anticorps anti-FHA.

Un conjugué immunogène comprenant un antigène couplé à une hémagglutinine filamenteuse de Bordetella pertussis ou une partie immunologiquement active de cette dernière ou à une hémagglutinine filamenteuse mutante à réaction immunologique croisée de Bordetella pertusis ou une partie de cette dernière est décrit dans EP 0 471 177.

Poulain-Godefroy et al. décrit la FHA de Bordetella pertussis dans des liposomes comprenant la glutathion-S-transférase de *Schistoma mansoni* (Sm 28 GST).

Aucune de ces publications ou brevets ne mentionne l'utilisation de la FHA comme adjuvant.

L'ensemble de ce qui précède indique que la FHA était utilisée pour ses propriétés de ciblage.

De manière surprenante, il a été montré selon la présente invention, que la protéine FHA, ou encore un polypeptide comprenant un fragment de la protéine FHA seule ou incluse dans une composition, est capable d'induire ou d'augmenter une réponse immunitaire à l'encontre d'un immunogène ou à l'encontre d'un antigène d'intérêt lorsque la FHA est présentée sous une forme libre, c'est-à-dire non physiquement liée à l'antigène d'intérêt contre lequel la réponse immunitaire est recherchée. Cela n'exclut pas une liaison physique avec toute molécule ou structure soit non antigénique, soit pour laquelle une éventuelle réponse immunitaire est indépendante de la réponse recherchée, par exemple dans le cadre d'une vaccination.

"Physiquement liée" signifie que la FHA ou son équivalent fonctionnel ne peuvent être administrés séparément de l'antigène d'intérêt. Cela inclut donc tout type de lien direct ou indirect avec un antigène d'intérêt. Par liaison directe, il est entendu que l'antigène d'intérêt et la FHA ou le fragment de la FHA sont liés par liaison covalente ou non covalente, mais sans intervention de substances, vecteurs, ou particules. Par liaison indirecte, il est au contraire entendu tout type d'association et/ou de liaison de la FHA ou du fragment de la FHA, de l'antigène d'intérêt avec une molécule, un vecteur, ou une structure quelconque ; lesdites molécule, vecteur ou structure peuvent comprendre, sans être limitatifs, des molécules transporteuses telle que la sérum albumine, des structures lipidiques tels des liposomes, des nanoparticules, des microsphères, des ISCOMs etc. Des liaisons indirectes peuvent également être réalisées par l'intermédiaire de liaisons covalentes, de liaisons électro-statiques ou des liaisons de type hydrophobe.

Un moyen de différencier la notion de "physiquement liée" à celle de "forme libre" qui caractérise les adjuvants de l'invention est la capacité des différents éléments de l'association (antigène d'intérêt, FHA ou fragment de la FHA, le cas échéant vecteur ou véhicule pharmaceutique) d'être séparés par des méthodes usuelles de séparation, telles la chromatographie, ou l'électrophorèse.

Il a ainsi été démontré selon l'invention, que l'utilisation de la protéine FHA ou d'une hémagglutinine filamenteuse ayant au moins 70% de similarité des séquences avec la FHA de *B. pertussis* et augmentant la réponse immunitaire spécifique d'un antigène sous forme libre pour la préparation des adjuvants était capable d'induire la production d'anticorps sériques ainsi que la production au niveau des muqueuses d'anticorps spécifiques de cet antigène ou immunogène. La FHA n'est pas physiquement liée au sens défini ci-dessus aux autres antigènes ou immunogènes.

Les antigènes sont immunogènes vis à vis de l'antigène contenu dans celle-ci et non vis-à-vis de la FHA.

Le demandeur a aussi montré que, de manière surprenante, la protéine FHA possédait en tant que telle une activité adjuvante de la réponse immunitaire, lorsqu'elle est présentée sous une forme libre, c'est-à-dire non physiquement liée à l'antigène d'intérêt contre lequel la réponse immunitaire est recherchée.

Par adjuvant au sens de la présente invention, on entendra un adjuvant apte à induire ou à augmenter la réponse immunitaire spécifique vis-à-vis d'un antigène ou d'un immunogène, ladite réponse immunitaire consistant indifféremment en une réponse humorale et/ou cellulaire. Cette réponse immunitaire consiste généralement en une stimulation de la synthèse d'immunoglobulines spécifiques d'un antigène donné, en particulier des IgG, IgA, IgM.

De façon encore plus surprenante, il a été démontré que cette activité adjuvante se manifestait avec efficacité lors des administrations des compositions par voie mucosale.

Un premier objet de l'invention consiste donc en l'utilisation de la protéine FHA ou d'une hémagglutine filamenteuse ayant au moins 70% de similarité des séquences avec la FHA de *B. pertussis* telle' que définie plus haut, sous forme libre, pour la préparation d'un adjuvant de la réponse immunitaire.

Toute protéine, telle que la FHA de B. bronchiseptica ou celle de *B.* parapertussis qui présente au moins 70 % de similarité avec la FHA de *B. pertussis* peut également être utilisé pour la préparation d'un adjuvant et fait partie de l'invention.

Dans l'ensemble du texte, on entend par X% de similarité par rapport à une séquence de référence le fait que X % des acides aminés sont identiques ou modifiés par substitution conservative telle que définie dans le logiciel d'alignement des séquences d'acides aminés ClustalW (http:///bioweb.pasteur.fr/docs/doc-gensoft/clustalw//) et que (100-X) % peuvent être délétés, substitués par d'autres amino-acides, ou encore que (100-X) % peuvent être ajoutés à la séquence de référence.

Ces protéines sont considérées dans le présent texte comme des équivalents fonctionnels de la FHA quant aux propriétés adjuvantes de cette dernière.

Selon un autre aspect, l'invention concerne un adjuvant de la réponse immunitaire comprenant la protéine FHA ou d'une hémagglutinine filamenteuse ayant au moins 70% de similarité des séquences avec la FHA de *B. pertussis* et augmentant la réponse immunitaire spécifique d'un antigène, sous forme libre. Un tel adjuvant pourra être administrée à l'homme ou à l'animal simultanément ou séquentiellement à l'antigène d'intérêt à l'encontre duquel une réponse immunitaire est recherchée. Avantageusement, l'adjuvant selon l'invention sera administré simultanément à l'antigène d'intérêt. Elle peut également faire l'objet de plusieurs administrations, seule ou en association avec l'antigène ; en particulier, elle peut faire l'objet d'un traitement de rappel consécutif à une immunisation.

Cette activité adjuvante se manifeste non seulement lorsque l'adjuvant et l'antigène sont administrés conjointement dans une même composition, mais également quand l'antigène et l'adjuvant sont administrés de façon séparée dans le temps, soit par la même voie d'administration, soit par deux voies différentes. A titre d'exemple, l'antigène peut être administré par voie systémique et l'adjuvant par voie mucosale ou orale. De la même façon, le nombre d'administrations peut différer pour l'antigène et l'adjuvant. Selon l'antigène et la réponse immunitaire choisis, l'adjuvant peut être administré une seule fois et l'antigène plusieurs fois, ou l'inverse.

Par antigène au sens de la présente invention, on entendra toute molécule ou structure naturelle ou synthétique, quelle que soit sa nature (protéique, saccharidique ou encore lipidique etc.) reconnue par les cellules du système immunitaire et capable d'activer celles-ci de manière à induire une réponse immunitaire spécifique de cet antigène.

Il a ainsi été montré selon la présente invention, que la protéine FHA se comportait en tant que composé adjuvant capable d'initier ou d'accroître la réponse immunitaire à l'encontre de différents antigènes ou immunogènes de structures diverses, reconnues différemment par les cellules du système immunitaire.

L'objectif de l'addition d'un adjuvant à un antigène dans une composition est de provoquer ou stimuler la réponse immunitaire tant dans ses phases primaires (production d'IgM) que dans ses phases secondaires à savoir la production des IgG au niveau de l'immunité cellulaire systémique, ou des IgA au niveau de l'immunité mucosale. Celle-ci est particulièrement importante dans le cas de développement d'une immunité anti-infectieuse. En effet, l'adhérence des micro-organismes aux membranes des cellules épithéliales des muqueuses est la première étape de l'infection virale et de la colonisation bactérienne. Les anticorps de type IgA peuvent empêcher cette adhérence en recouvrant le micro-organisme (coating). Ils offrent ainsi une protection au niveau des sécrétions externes comme les larmes, la salive ou les sécrétions nasales ainsi qu'au niveau du mucus intestinal et pulmonaire. II est donc facile de concevoir que si la FHA selon l'invention produisent une stimulation de la réponse IgA, l'impact sur la mise au point de compositions immunogènes ou de vaccins est important.

Ainsi, une composition immunogène comprenant l'adjuvant selon l'invention et un immunogène ou un antigène est caractérisée en ce que le rapport en poids de l'adjuvant et de l'immunogène est compris entre 10⁻⁴ et 10⁴, de préférence entre 0,03 et 300 et de manière préférée entre 0,4 et 5.

Comme il a été dit plus haut, et démontré ci-après, l'adjuvant est administrable séparément de l'antigène dans le temps ou selon différentes voies d'administration, ou associés dans une composition immunogène.

Dans l'un et l'autre cas, l'antigène peut être d'origine bactérienne, virale ou parasitaire. II peut également s'agir d'un antigène spécifique de cellules cancéreuses, tel l'antigène de carcinome embryonnaire (ACE).

Quand l'antigène est d'origine bactérienne, il peut s'agir notamment d'antigènes de *Bordetella,* de *Shigella,* de *Neisseria,* de *Borrelia,* des toxines ou toxoïdes diphtériques, tétaniques ou cholériques.

Quand il est d'origine parasitaire, il peut par exemple être un antigène de *Plasmodium,* des *Schistosoma ou* de *Toxoplasma.*

Un autre objet de l'invention consiste en une composition vaccinale, caractérisée en ce qu'elle comprend une composition adjuvante telle que décrite ci-dessus, en association avec un véhicule phal-maceutiquement compatible.

La préparation de compositions vaccinales contenant un polypeptide en tant que molécule immunogène ou antigénique est bien connue de l'homme du métier et est en particulier illustrée dans les brevets US N°4,608,251; 4,601,903; 4,599,231; 4,599,230; 4,596,792 et 4,578,770.

La FHA et l'antigène d'intérêt peuvent être formulés, au sein d'une composition vaccinale selon l'invention, sous une forme neutre ou saline.

Des sels pharmaceutiquement compatibles comprennent des sels d'addition acide (formés avec les groupes amino libres du peptide) ou encore ceux formés avec des acides inorganiques tels que par exemple les acides chlorhydrique et phosphorique ou encore ceux formés avec des acides organiques tels que l'acide acétique, oxalique, tartrique et mandélique.

Des sels formés avec les groupes carboxy libres peuvent également être dérivés de bases inorganiques telles que par exemple des hydroxydes de sodium, potassium, ammonium, calcium ou ferrique, ou encore ceux formés avec des bases organiques telles que l'isopropylamine, la triméthylamine, le 2-éthylaminoéthanol, l'histidine, ou encore la procaïne.

L'invention porte enfin sur un médicament pour le développement des défenses immunitaires d'un organisme et comprenant la FHA ou d'une hémagglutinine ayant au moins 70% de similarité des séquences avec la FHA de *B. pertussis* et augmentant la réponse immunitaire spécifique d'un antigène à titre de principe actif.

Les résultats décrits dans la partie expérimentale ci-après ont été obtenus par administration par voie nasale de souris avec un adjuvant ou avec un immunostimulant selon l'invention. Lorsqu'une composition immunogène comprenant sous forme non liée la FHA ou un équivalent fonctionnel de celle-ci est un antigène, les trois antigènes utilisés dans des conditions expérimentales décrites ci-après sont la Sm28 GST décrite ci-avant, et l'hémocyanine de *Megathura Crenulata* (KLH).

Les inventeurs ont ainsi mis en évidence que l'administration nasale de l'antigène avec la FHA présentait les propriétés suivantes :
a) dans des conditions où l'antigène seul n'est pas capable d'induire une réponse sérique significative, la présence de FHA a permis d'induire l'induction d'une réponse immune spécifique chez 7 souris sur 9. De plus, l'induction d'une réponse immune dirigée contre l'antigène n'est pas corrélée avec une réponse dirigée contre la FHA.
b) l'analyse des anticorps présents dans les liquides de lavage broncho-alvéolaires (BALF) indique de manière surprenante que la FHA entière a un effet adjuvant sur la production d'IgA non spécifiques de l'antigène au niveau broncho-alvéolaire alors qu'il n'y a pas d'effets significatifs sur la quantité totale d'IgG.
c) les résultats exposés ci-après indiquent que cette activité adjuvante de la FHA s'exprime au moins vis-à-vis de deux antigènes différents.
   En outre, cette activité adjuvante persiste par voie systémique : des souris immunisées par voie sous-cutanée par l'antigène seul ou l'antigène et l'adjuvant de l'invention deux fois à deux semaines d'intervalle répondent mieux dans le cas où l'adjuvant est présent.
d) si des souris préalablement vaccinées, par exemple par le DTCoq, sont immunisés par voie nasale deux mois plus tard avec un antigène et l'adjuvant de l'invention deux fois à deux semaines d'intervalle, il apparaît que la vaccination Dtcoq qui provoque l'apparition d'anticorps anti-FHA, n'a pas empêché l'induction d'une réponse dirigée contre l'antigène.

Les résultats exposés plus en détails ci-après montrent à l'évidence que l'adjuvanticité de la FHA libre est une activité intrinsèque de cette molécule, qui est indépendante d'une liaison physique à l'antigène au sens défini ci-avant ; autrement dit, l'activité adjuvante de cette molécule n'est pas liée à une fonction de vectorisation de l'antigène par la FHA. En d'autres termes, la FHA peut constituer un adjuvant dans des compositions immunogènes ou dans des vaccins et ne possède aucune action de ciblage de l'antigène vers les cellules immuno-compétentes.

La FHA constitue donc un nouvel adjuvant efficace par voie mucosale représentant une alternative avantageuse aux adjuvants de l'état de la technique, qu'il s'agisse d'adjuvants sous forme particulaire tels que les liposomes, les microsphères, les ISCOMs qui sont tous des structures globulaires synthétiques encapsulant l'antigène administré, ou encore aux micro-organismes recombinants exprimant un antigène d'intérêt.

En outre, la FHA se caractérise par une totale innocuité et ne provoque pas la sécrétion de cytokines proinflammatoires au niveau local, au contraire des adjuvants muqueux les plus efficaces actuellement connus que sont les toxines bactériennes, telles que la toxine cholérique, la toxine de E. coli et la toxine de *Bordetella pertussis.*

L'identification selon l'invention de la FHA ou d'un équivalent fonctionnel de celle-ci en tant qu'adjuvant de la réponse immunitaire, notamment de la réponse immunitaire mucosale, vient donc combler un réel besoin dans l'état de la technique, d'un adjuvant muqueux efficace et dépourvu de toute toxicité.

De plus, l'activité adjuvante de la FHA n'est pas restreinte ou dépendante d'un haplotype donné d'antigène d'histocompatibilité. Au contraire, l'adjuvanticité de ces protéines est observée quel que soit l'haplotype de MHC, comme le démontrent les expériences réalisées sur des souris génétiquement hétérogènes, comme les souris "Outbred ", telles que les souris OF1.

Une analyse des différents isotypes d'anticorps sériques produits en réponse à l'immunisation contre ces deux antigènes en présence de FHA a montré que le rapport quantitatif entre les différents isotypes d'IgG n'était pas significativement différent selon que l'antigène était administré simultanément à la FHA.

Par ailleurs, l'analyse des isotypes d'anticorps sériques montre une prédominance d'isotypes IgG1 et IgG2a en réponse à l'administration de la Sm28GST ou de la KLH en présence de FHA.

Ces résultats indiquent que le profil isotypique des anticorps spécifiques produits dépend essentiellement de l'antigène utilisé, la FHA n'induisant pas, par elle-même, de polarisation.

L'ensemble des résultats obtenus et exposés en détail dans la partie expérimentale démontre que la FHA peut être utilisée comme adjuvant en tant que tel, quel que soit l'haplotype d'histocompatibilité de l'individu à immuniser, et quelle que soit la nature de l'antigène ou de l'immunogène à l'encontre duquel une réponse immunitaire de type mucosal et/ou systémique est recherchée.

Un tel polypeptide pourra être obtenu par recombinaison génétique conformément à l'enseignement de Brown et al. (2), de Relman et al, (3) ou encore de Delisse-Gathoye (4).

Dans un mode particulier de réalisation de la protéine FHA ou d'une hémagglutinine filamenteuse ayant au moins 70% de similarité des séquences avec la FHA de *B. pertussis* et augmentant la réponse immunitaire spécifique d'un antigène selon l'invention, un polynucléotide codant la FHA ou d'une hémagglutinine filamenteuse ayant au moins 70% de similarité des séquences avec la FHA de *B. pertussis* et augmentant la réponse immunitaire spécifique d'un antigène est inséré dans un vecteur d'expression comprenant au moins un promoteur et un terminateur nécessaires à l'expression du polynucléotide dans une cellule hôte appropriée.

Le polynucléotide codant un équivalent fonctionnel de la FHA au sens défini ci-avant a au moins 70 % de similarité avec la séquence codant la FHA et décrite dans (2).

Par similarité, on entend le fait que, pour un même cadre de lecture, un triplet donné est traduit par le même acide aminé. Ce terme inclut donc les modifications de bases résultant de la dégénérescence du code génétique.

Le pourcentage de similarité est déterminé en comparant une séquence donnée avec la séquence de référence. Lorsque celles-ci sont de longueurs différentes, le pourcentage de similarité est basé sur le pourcentage de nucléotides de la séquence la plus courte similaires à ceux de la séquence la plus longue.

Le degré de similarité peut être déterminé conventionnellement par utilisation de logiciels tels le ClustalW (Thompson et al., Nucleic Acids Research 22 (1994), 4673-4680) distribués par Julie Thompson (Thompson@EMBL-Heidelberg.DE) et Toby Gibson (Gibson@EMBL-Heidelberg.DE) du Laboratoire Européen de Biologie Moléculaire, Meyerhosfstrasse 1, D 69117 Heidelberg, Germany. ClustalW peut aussi être chargé à partir de plusieurs sites web incluant IGBMC (Institut de Génétique et de Biologie Moléculaire et Cellulaire, B.P. 163, 67404 Illkirch cedex France; ftp://ftp-igbmc.u-strabg.fr/pub/) et EBI (ftp://ftp.ebi.ac.uk/pub/software/) et tous les sites renvoyant à l'Institut de Bioinformatique, Wellcome Trust Genome Campus, Hinxton, Cambridge CB10 1SD, UK).

Un vecteur d'expression comprenant de tels polynucléotides comprendra en outre avantageusement au moins une origine de réplication fonctionnelle dans la cellule hôte dans laquelle l'expression de la protéine FHA ou d'une partie de la protéine FHA recombinante est recherchée, ainsi qu'au moins un marqueur de sélection tel qu'un marqueur de résistance à un antibiotique, par exemple la néomycine, la tétracycline, la rifampycine ou l'ampicilline .

Une cellule hôte appropriée peut être indifféremment d'origine bactérienne ou eucaryotique.

Pour construire de tels vecteurs d'expression et transformer ou transfecter des cellules hôtes appropriées, l'homme du métier pourra se référer avantageusement à l'ouvrage de Sambrook et al. (2001), Molecular Cloning: A Laboratory Manual. 3^{ème} Edi. Cold Spring Harbor Laboratory, Cold Spring Harbor, New York.

La purification de la protéine FHA recombinante ou d'une hémagglutinine filamenteuse ayant au moins 70% de similarité des séquences avec la FHA de *B. pertussis* et augmentant la réponse immunitaire spécifique d'un antigène peut être réalisée par des techniques bien connue de l'homme du métier.

La protéine FHA peut également être préparée par des procédés classiques de synthèse chimique, indifféremment en solution homogène ou en phase solide.

A titre illustratif de tels procédés de synthèse chimique polypeptidique, l'homme du métier pourra se référer à l'enseignement de Houbenweyl (1974), In Methode der Organischen Chemie, E. Wunsh Ed., Vol.15-I et 15-II, Thieme, Stuttgart ou encore la technique de Merrifield (1965a;1965b); Merrifield RB, (1965a), Nature, 207(996): 522-523, Merrifield (1965b), Science, 150(693):178-185.

La protéine FHA telle que précédemment définie peut également être utilisée pour la préparation d'une composition adjuvante d'une réponse immunitaire lorsque certains acides aminés sont substitués par substitution conservative. Par substitution conservative, on entend la substitution d'un acide aminé par un autre n'ayant aucune conséquence ou des conséquences mineures sur la structure tertiaire de la séquence et sur les caractères d'hydrophobicité de la même séquence. A titre d'exemple, la substitution d'une guanine par une alanine ou vice-versa est qualifiée de substitution conservative. De la même façon, la valine, la leucine, l'isoleucine sont des acides aminés qui peuvent être substitués mutuellement de façon conservative. D'autres groupes de substitution conservative peuvent être, sans être limitatifs, (D,E), (K,R), (N,Q), (F,W, Y).

Dans un mode particulier de réalisation d'un polypeptide contenant tout ou partie de la protéine FHA mature, ledit polypeptide pourra avantageusement être rendu résistant à la protéolyse, par exemple par remplacement de la liaison peptidique -CONH- par une liaison réduite - CH₂NH-, une liaison rétro-inverso -NHCO-, une liaison méthylène-oxy -CH₂O-, une liaison thiométhylène -SH₂-S-, une liaison carba -CH₂CH₂-, une liaison cétométhytène -CO-CH₂-, une liaison hydroxyéthylène -CHOH-CH₂-, une liaison -N-N, une liaison E-alcène ou encore une liaison -CH=CH-.

Selon un autre mode de réalisation, le polypeptide comprenant tout ou partie de la séquence d'acides aminés de la protéine FHA, un ou plusieurs résidus d'acides aminés de la forme L pourront être remplacés par l'acide aminé correspondant sous la forme D; un résidu acide glutamique pourra être remplacé par un résidu d'acide pyro-glutamique. La synthèse de peptides contenant au moins un résidu d'acides aminés sous la forme D est par exemple décrite par Koch Y., (14).

Selon un autre aspect, la composition immunogène selon l'invention est caractérisée en ce que l'antigène d'intérêt est d'origine bactérienne, virale ou parasitaire, animale, végétale ou humaine.

La présente invention est en outre illustrée, sans pour autant être limitée, par les figures et les exemples suivants :
La figure 1 illustre les titres d'anticorps sériques spécifiques de la protéine Sm28GST ou de la FHA, après immunisation de souris contre la protéine Sm28GST en présence ou en l'absence de FHA.
La figure 2 représente les densités optiques observées pour une dilution de ¼ en anticorps isotypes IgA et IgG dans les fluides de lavage bronchoalvéolaire de souris immunisées contre la Sm28GST en présence ou en absence de FHA.
La figure 3 représente les densités optiques observées pour une dilution de 1/80 en anticorps totaux d'isotypes IgA et IgG dans les fluides de lavage bronchoalvéolaires de souris immunisées avec la protéine Sm28GST en présence ou en absence de FHA.
La figure 4 illustre le profil isotypique des anticorps sériques spécifiques de la Sm28GST chez des souris immunisées contre cette protéine en présence ou en absence de FHA.
La figure 5 illustre une élution sélective de la FHA et de la protéine Sm28GST à partir d'un mélange de l'adjuvant et de cet antigène.
La figure 6 illustre la réponse spécifique anti Sm28GST après vaccination avec le Dtcoq.
La figure 7 illustre les titres en anticorps obtenus après administration par voie sous-cutanée de l'antigène Sm28GST avec ou sans la FHA après immunisation deux fois à deux semaines d'intervalle. L'analyse de la réponse sérique a été observée une semaine plus tard.
La figure 8 représente l'effet adjuvant et immuno-stimulant de la FHA, après administration par voie orale à trois groupes de souris ayant reçu à deux semaines d'intervalle :
   - 30 µg de KLH,
   - 30 µg de KLH + 5 µg de FHA entière.
La figure 8a représente la densité optique à J21 observées en IgG1, IgG2a et IgG2b spécifiques de la KLH dans les sérums pour les dilutions respectives de 1/800, 1 /100 et 1/100.
La figure 8b représente la densité optique à J21 observées en IgG totaux non spécifiques dans les sérums pour une dilution de 1/10000.
La figure 8c représente la densité optique à J21 observées en IgA, totaux non spécifiques dans les liquides de lavages intestinaux pour une dilution de 1/800.
La figure 9 représente l'analyse des mRNA induits localement au niveau du poumon après administration soit de solution saline apyrogène, soit de 5 µg de FHA, soit de 20 µg de LPS.

### EXEMPLES:

### A. MATERIELS ET METHODES

### • Antigènes

La glutathion S-transférase de *Schistosoma mansoni* (Sm28GST) a été fournie par la Société Transgène (Strasbourg, France). La FHA a été purifiée par chromatographie sur une colonne d'héparine-Sépharose, comme décrit précédemment (Menozzi et al. FEMS, 1991) à partir des surnageants de *B.* pertussis BPRA *[Bordetella pertussis* RA], une souche dépourvue du gène de la toxine pertussique.

La préparation de FHA a été éluée sur une colonne d'Acticlean [Stérogène] pour éliminer les contaminants endotoxiniques. L'activité endotoxinique finale a ensuite été évaluée par un test Limulus (Biowhittaker). L'hémocyanine de *Megathura crenulata* (KLH) a été achetée chez Calbiochem.

### • Immunisations

Des souris OF1 de 6 semaines (Iffa Credo, L'Arbresle, France) ont été anesthésiées par voie intrapéritonéale avec 200 µl de pentobarbital sodique (5%; Sanofi, Libourne, France) pour 10 g de poids corporel. Les souris ont été immunisées par voie nasale avec 40 µl de solution saline apyrogène dans laquelle ont été diluées les différentes préparations antigéniques. Les souris ont été instillées deux fois à deux semaines d'intervalle et sacrifiées une semaine plus tard pour pouvoir effectuer les prélèvements sanguins et les lavages broncho-alvéolaires. Les doses d'antigène utilisées sont décrites dans les exemples.

### • Recueil et analyse des échantillons

Les souris ont été saignées à la queue (ou par ponction cardiaque le jour du sacrifice) et les sérums ont été conservés à -20°C jusqu'au jour de l'analyse. Les liquides de lavages bronchoalvéolaires (BALF) ont été collectés par cannulation de la trachée et résultent d'un lavage des poumons avec 0,5 ml de PBS. Après centrifugation de 10 mn à 4000g pour éliminer les cellules et les débris tissulaires, les BALF ont ensuite été congelés à 20°C après adjonction de fluorure de phényl-méthyl-sulfonyle à la concentration finale de 1 mM .

Les taux d'anticorps dans le sérum et dans les BALF ont été déterminés par ELISA. Les microplaques (Maxisorp, Nunc) ont été incubées pendant toute la nuit à 4°C avec 50 µl/puits d'une solution de FHA (5µg/ml) ou de Sm28GST (10 µg/ml), ou de KLH (5 µg/ml). Les sérums dilués dans du PBS contenant 1%o de Tween-20 et 5%o de gélatine (PBS/Tw/g), ont alors été ajoutés dans les plaques après 4 rinçages avec du PBS contenant 1%o de Tween-20 (PBS/Tw). Après incubation pendant une nuit à 4°C, les plaques ont été rincées 4 fois avec du PBS/Tw puis incubées avec différentes dilutions d'anticorps (dans du PBS/Tw/g; 1h30 à 37°C) conjugués à la peroxydase (anti-souris IgG (H+L) ou IgG1, ou IgG2a, ou IgG2b ou IgG3; Southern Biotechnologies Associates, Birmingham, USA). Après 4 rinçages avec du PBS/Tw, les plaques ont été révélées avec une solution à 1 mg/ml d'ABTS (Sigma) dans du tampon citrate (0,1 M pH 4) et contenant 0,03% d'H₂O₂. La densité optique a été mesurée à 405 nm (Titertek Multiscan MCC/340) au bout de 30 minutes. Les anticorps d'isotype IgA ont été détectés après incubation avec un anticorps anti-IgA de souris biotinylé (Zymed) dilué dans du PBS/Tw/g pendant 1 h30 à 37°C. Après 4 rinçages avec du PBS/Tw, les plaques ont été incubées pendant 30 minutes à 37°C en présence de streptavidine couplée à la peroxydase (Amersham) diluée dans du PBS/Tw/g. Après 6 rinçages avec du PBS/Tw, les plaques ont été révélées avec une solution d'OPD (1mg/ml; Sigma) pendant 30 minutes à 37°C. La réaction a été stoppée avec une solution d'HCl 2N et la densité optique a été mesurée à 492 nm. La droite de régression donnant le logarithme de la densité optique observée en fonction de l'inverse de la dilution a été calculée. Les titres correspondent, par extrapolation de cette droite, à l'inverse de la dilution pour laquelle la valeur de la densité optique est égale à trois fois la valeur de la densité optique obtenue avec du PBS.

La détection des anticorps dans les BALF s'effectue de la même façon avec quelques modifications. Après l'adsorption de l'antigène, les plaques sont saturées avec une solution de gélatine (5%o en PBS) pendant 30 minutes à température ambiante. Les BALF et les anticorps sont ensuite dilués avec du PBS/Tw. Les concentrations d'IgA totales et d'IgG totales dans ces BALF ont été évaluées sur des microplaques dans lesquelles ont été préalablement adsorbés des anticorps anti-souris IgA ou IgG non marqués et comparaison avec une gamme étalon réalisée avec de l'IgA ou de l'IgG myélomateux de souris purifié (Sigma).

### • Test de Liaison

Des billes de Sépharose couplées à l'héparine (CL-6B; Pharmacia) ont été resuspendues dans du PBS et tassées dans une colonne (diamètre 1 cm pour 1,3 ml de gel). Après rinçage de cette colonne, une solution contenant de la Sm28GST (200 µg) et de la FHA (40 µg) a été déposée.

Après rinçage, des concentrations croissantes de NaCl ont été déposées sur cette colonne (de 0,1 M NaCl jusqu'à 1M). L'analyse des échantillons recueillis après passage sur la colonne puis élution, a été effectuée, après précipitation au TCA, par électrophorèse sur gel d'acrylamide.

### • Détermination des ARN messagers

Les souris sont immunisées comme précédemment, par voie nasale (volume = 50 µl) soit avec du sérum physiologique apyrogène, soit avec 5 µg de FHA, soit avec 20 µg de LPS (Sigma). Des souris non administrées sont utilisées comme témoin. A différentes périodes de temps (entre 1 h et 48 h) les souris sont sacrifiées, les poumons entiers sont prélevés et broyés dans une solution de RNAzol®. Les ARN sont extraits à l'aide de chloroforme puis précipités par l'isopropanol. Après lavage, le culot d'ARN est remis en suspension dans l'eau. Une transcription réverse est réalisée et permet de synthétiser l'ADNc correspondant aux ARN extraits. Sur tous nos extraits des expériences de polymérisation en chaîne (PCR) ont été réalisées et ont permis l'amplification de fragments d'ADN spécifique de différents marqueurs avec les couples d'amorces indiqués dans le tableau ci-dessous :

| CYTOKINE | SEQUENCES |
|---|---|
| IL1 Ra | sens 5' AGA CCC TGC AAG ATG CAA GCC TTC AGG 3' |
| | anti-sens 5' GGT CAG CCT CTA GTG TTG TGC AGA 3' |
| IL6 | sens 5' GTG ACA ACC ACG GCC TTC CCT ACT 3' |
| | anti-sens 5' GGT AGC TAT GGT ACT CCA 3' |
| IL10 | sens 5' CGG GAA GAC AAT AAC TG 3' |
| | anti-sens 5' CAT TTC CGA TAA GGC TTG G |
| IL12 | sens 5' GAC CCT GCC CAT TGA ACT GGC 3' |
| | anti-sens CAA CGT TGC ATC CTA GGA TCG 3' |
| TNFα | sens 5' AGC CCA CGT CGT AGC AAA CCA CCA A 3' |
| | anti-sens 5' ACA CCCATT CCC TTC ACA GAG CAA T 3' |
| MHC II | sens 5' TGT CCA GGA CAG AGG CCC TC 3' |
| | anti-sens 5' TCC ACA TGG CAG GTG TAG AC 3' |
| B7-1 | sens 5' GTA TTG CTG CCT TGC CGT TA 3' |
| | anti-sens 5' ATG GTG TGG TTG CGA GTC GT 3' |
| B7-2 | sens 5' AGG ACA TGG GCT CGT ATG AT 3' |
| | anti-sens 5' GAA CAC ACA CAA CGG TCA TA 3' |

Les produits d'amplification ont été visualisés par utilisation de bromure d'éthidium après migration sur un gel d'agarose. Les bandes obtenues ont été analysées par une technique d'analyse d'image et un index correspondant à l'intensité de ces bandes a été établi.

### EXEMPLE 1

### Etude de l'activité adjuvante de la FHA vis-à-vis d'une réponse immunitaire à l'encontre de la protéine Sm28GST de Schistosoma mansoni.

Des souris OF1 (" Outbred ") ont reçu une administration par instillation nasale avec 50µg de Sm28GST, en présence ou non de 5 µg de FHA dilués dans le même échantillon, deux fois à deux semaines d'intervalle.

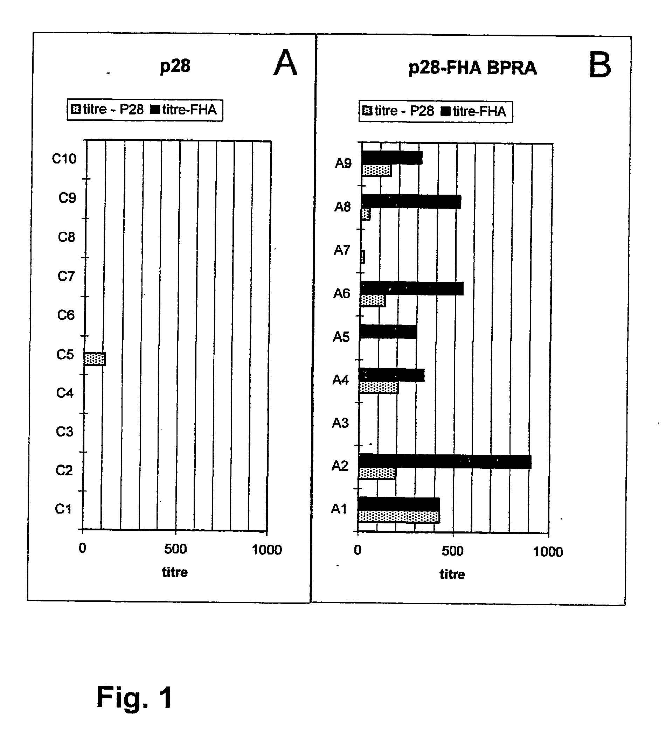

La production d'anticorps sériques d'isotype IgG sérique spécifiques de la Sm28GST ou de la FHA une semaine après la deuxième instillation nasale a été analysée et les résultats sont reportés dans la figure 1.

Le titre en anticorps IgG sériques dirigés contre la Sm28 (barres grisées) et la protéine FHA (barres noires) a été mesuré dans le sérum de souris ayant reçu respectivement la protéine Sm28GST (figure 1A), un mélange Sm28GST + FHA (figure 1B).

Ces résultats indiquent que la protéine Sm28GST seule n'est pas capable d'induire une réponse en anticorps sérique significative. En revanche, la présence de FHA a permis l'induction d'une réponse immunitaire spécifique contre la Sm28GST chez sept souris sur neuf. En outre, l'analyse de la réponse dirigée contre la FHA chez ces animaux montre que l'induction d'une réponse immune dirigée contre la Sm28GST n'est pas nécessairement corrélée avec une réponse spécifique dirigée contre la FHA.

### EXEMPLE 2 :

### Etude de l'activité adjuvante de la FHA sur la Production d'anticorps par les cellules immunitaires du tractus respiratoire.

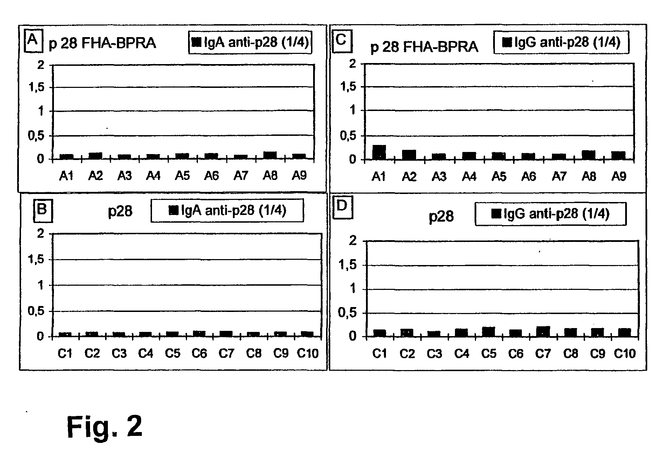

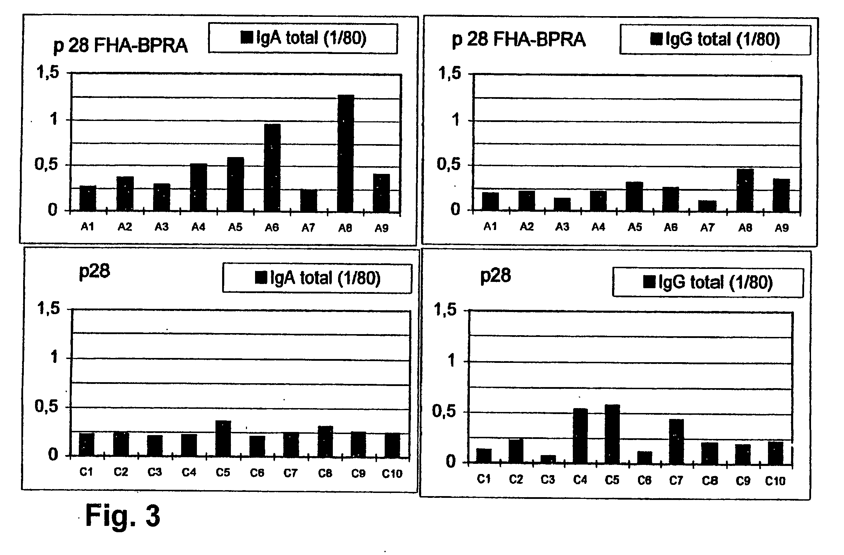

Des souris OF1 ont été immunisées selon le protocole décrit à l'exemple 1 et la production d'anticorps d'isotypes IgG et IgA totaux ou spécifiques de la protéine Sm28GST contenus dans les liquides de lavage bronchoalvéolaires ont été analysés. Les résultats sont reportés dans les figures 2 et 3.

Les titres en anticorps spécifiques de la protéine Sm28GST (figure 2) ou en anticorps totaux (figure 3) dans les liquides de lavage bronchoalvéolaires d'isotype IgA (A, B) ou IgG (C, D) ont été mesurés chez des souris, 21 jours après la seconde instillation nasale de Sm28GST (B, D), de Sm28GST + FHA (A, C).

Les résultats de la figure 2 indiquent que la présence de la FHA lors de l'immunisation n'a pas induit une production détectable d'anticorps spécifiques de la Sm28GST dans les liquides de lavage bronchoalvéolaires.

Les résultats de la figure 3 montrent que l'on peut observer un effet notable de la FHA sur la quantité d'anticorps totaux d'isotype IgA contenus dans les liquides de lavage bronchoalvéolaires, alors qu'il ne semble pas y avoir d'effet significatif sur la quantité totale d'anticorps d'isotype IgG.

### EXEMPLE 3 :

### Etude de la polarisation de l'activité adjuvante de la FHA: réponse isotypique.

Les protocoles d'immunisation sont identiques à ceux des exemples 1 et 2, des instillations nasales ayant été réalisées chez les souris avec un mélange contenant respectivement 50 µg d'antigène et 5 µg de FHA.

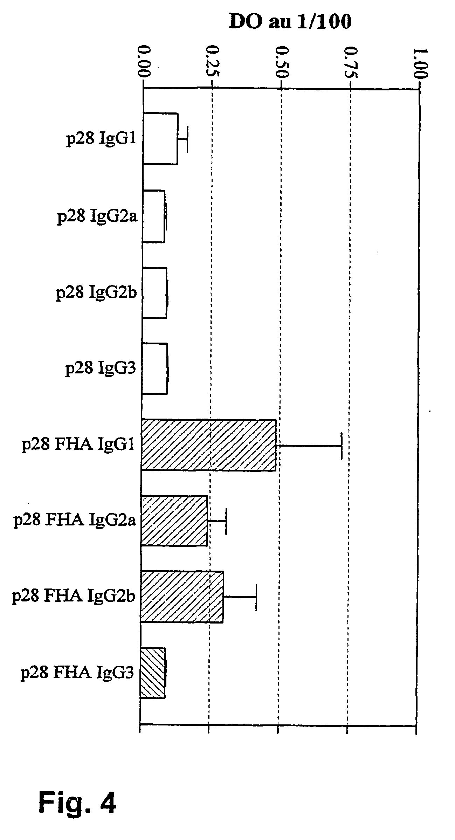

Les titres en anticorps sériques spécifiques d'un antigène ou immunogène d'intérêt ont été mesurés 21 jours après la seconde instillation nasale de souris ayant reçu respectivement cet antigène ou cet immunogène. La qualité des différents isotypes IgG1, IgG2a, IgG2b et IgG3 a été mesurée (figure 4).

L'antigène utilisé est la protéine Sm28GST de *Schistosoma mansoni.*

Les résultats de la figure 4 montrent, comme cela a déjà été montré dans les exemples 1 et 2, l'activité adjuvante de la FHA entière. L'analyse du profil des différents isotypes d'anticorps IgG montre une production quantitativement similaire d'anticorps d'isotypes IgG1 et IgG2a, spécifiques de la protéine Sm28GST, significative d'une réponse immunitaire dite " mixte ".

### EXEMPLE 4 :

### La FHA et l'antigène ne sont pas physiquement liés dans une composition immunogène selon l'invention.

Afin de déterminer l'existence d'une liaison physique non covalente entre l'antigène d'intérêt de la FHA, de nature à constituer un complexe, un mélange de FHA et de Sm28GST a été préparé comme indiqué dans la partie " Matériels et Méthodes".

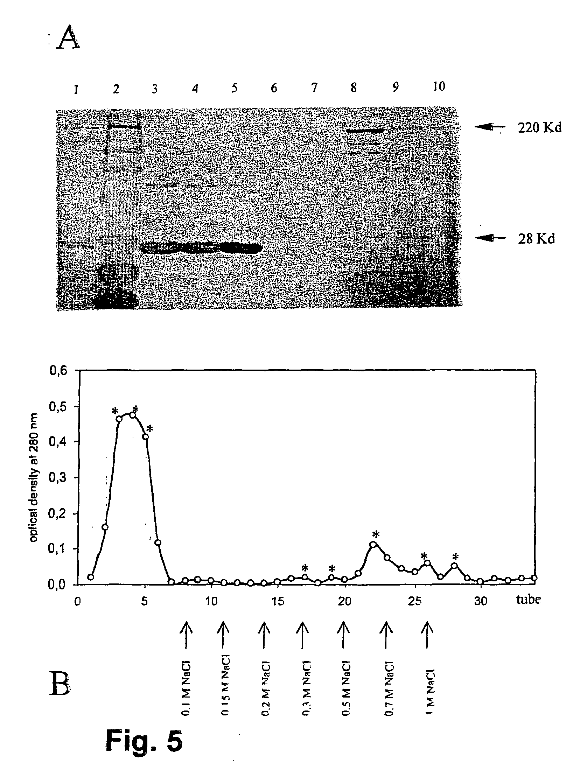

Le mélange FHA et Sm28GST a été déposé sur une colonne d'héparine. Une élution a été réalisée avec des concentrations croissantes de NaCl, chacune des fractions d'élution ayant ensuite été analysée pour sa concentration en protéines (figure 5b).

Des fractions ont également été soumises à une migration électrophorétique dans un gel de polyacrylamide en présence de SDS simultanément à une série de marqueurs de poids moléculaire protéique (figure 5a).

Les résultats montrent que l'élution dans un gradient croissant de NaCl a permis de désorber la FHA dès une concentration de 0,5 M de NaCl (voir figure 5b). Par ailleurs, l'analyse de la nature des protéines présentes dans les différentes fractions d'élution a permis de montrer qu'aucune trace de la protéine Sm28GST n'était détectée (figure 5a).

En conséquence, il est ici montré une absence totale d'interaction physique covalente ou non covalente entre la FHA et l'antigène d'intérêt.

### EXEMPLE 5 :

### L'activité adjuvante de la FHA persiste chez les sujets vaccinés.

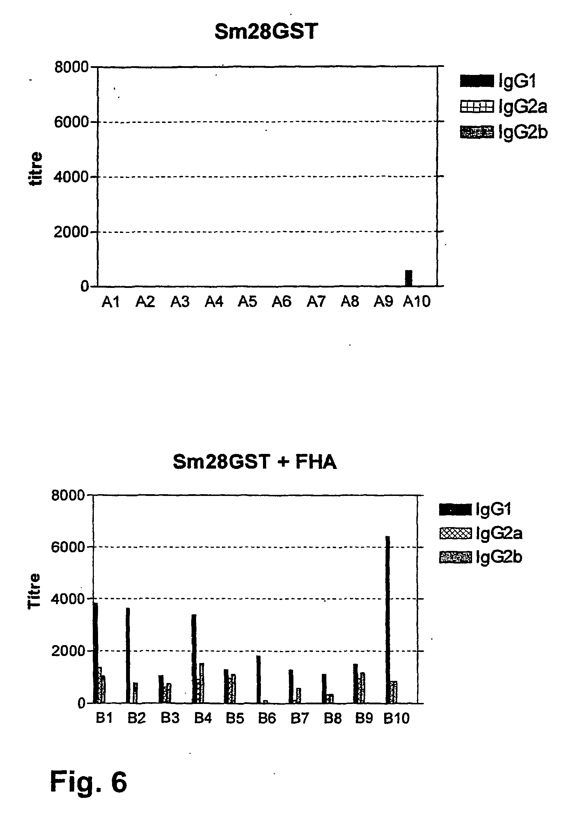

Des souris OF1 ont été vaccinées par injection sous-cutanée de 50 µl de gène Dtcoq (vaccin commercial). Deux mois plus tard elles ont été immunisées par voie nasale, deux fois à deux semaines d'intervalle. L'analyse de la réponse sérique obtenue une semaine plus tard, montre que la vaccination par le Dtcoq, qui provoque l'apparition d'anticorps circulant anti-FHA n'a pas empêché l'induction d'une réponse dirigée contre la Sm28GST dans le groupe co-administré avec de la FHA (fig. 6).

Les expériences des exemples 5 et 6 confirment le pouvoir adjuvant intrinsèque de la FHA, par une voie systémique et son utilisation potentielle dans les populations vaccinées.

### EXEMPLE 6 :

### L'activité adjuvante de la FHA persiste par voie systémique.

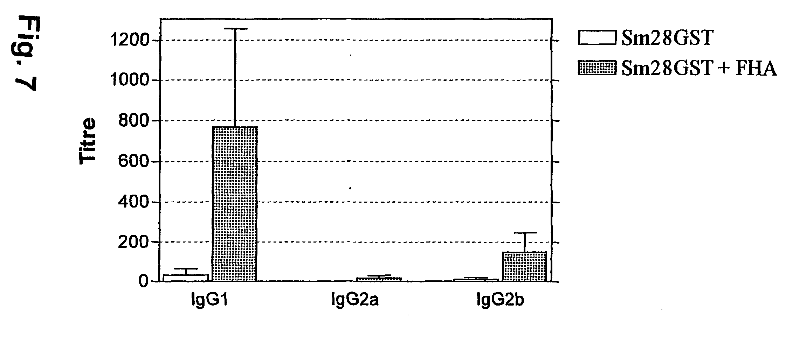

Des souris OF1 ont été immunisées par voie sous-cutanée soit avec 50 µg de SmGST, deux fois à deux semaines d'intervalle. L'analyse de la réponse sérique obtenue une semaine plus tard, montre l'induction d'une réponse dirigée contre la Sm28GST dans les deux groupes qui ont été co-administrés avec de la FHA (fig. 7). Cette réponse reste cependant plus faible que celle qui avait été obtenue par voie nasale.

### EXEMPLE 7 :

### L'activité adjuvante de la FHA persiste par voie orale.

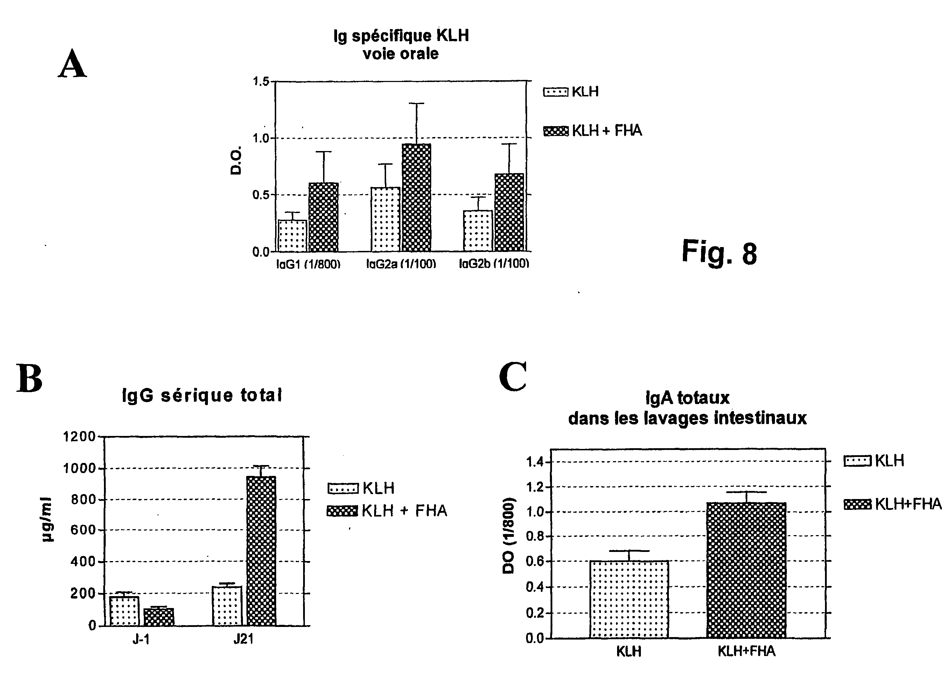

Des souris OF1 ont été immunisées par voie orale soit avec 30 µg de KLH, soit avec 30 µg de KLH et 5 µg de FHA, deux fois à deux semaines d'intervalle. L'analyse de la réponse sérique obtenue une semaine plus tard, montre l'induction d'une réponse dirigée contre la KLH dans le groupe co-administré avec de la FHA (Figure 8A).

Pour l'administration par voie orale, la ou les protéines sont mises en solution à la concentration requise dans une solution de PBS contenant 30 g/l de NaHCO₃. L'administration d'un volume de 200 µl se fait sur les animaux non anesthésiés à l'aide d'une sonde gastrique.

Les lavages intestinaux sont effectués à J21 après rupture cervicale des animaux selon une modification de la technique décrite par Nedrud et coll. (1987). L'intestin est sectionné sous l'estomac et au-dessus du caecum et rincé dans le PBS. Puis il a été fendu sur toute la longueur. L'intestin et son contenu ont été re-suspendus dans 2 ml du tampon suivant 25 mM NaCl, 40mM, Na₂SO₄, 10mM KCL, 20 mM NaHCO₃, 50mM EDTA, 162 mg/ml de polyethylene glycol (M.W. 3350) et 1 ‰ d'aprotinine. Après centrifugation les surnageants sont congelés après addition de 1 mM PMSF.

A part ce point particulier, les conditions de matériel et méthodes en particulier pour les dosages d'anticorps, sont identiques à celles précédemment citées.

### EXEMPLE 8 :

### La FHA peut provoquer une activation polyclonale des plasmocytes ce qui génère une production d'anticorps.

### 8.1. Administration par voie nasale :

La figure 3 de l'exemple 2 illustre les taux d'anticorps totaux non spécifiques détectés dans les liquides de lavage brocho-alvéolaires après administration par voie nasale de Sm28GST en présence ou non de FHA ou de FHA44.

Comme il était précisé plus haut, les résultats de la figure 3 montrent que la FHA44 induit des taux massifs d'anticorps d'isotypes IgA et IgG totaux dans les sécrétions bronchoalvéolaires qui ne peuvent être uniquement corrélés à l'apparition des anticorps spécifiques.

### 8.2 Administration par voie orale :

L'analyse des taux d'immunoglubulines totaux des prélèvements obtenus lors de l'expérience d'administration par voie orale a été effectuée. On observe une nette augmentation des IgG totaux sériques dans le groupe qui a reçu de la FHA (Figure 8 B). De plus, la FHA a été capable d'augmenter les taux d'IgA non spécifiques dans les liquides de lavage intestinal (Figure 8 C) alors même qu'aucune réponse spécifique n'y a été détectable.

Cette activité non spécifique de la FHA et de ses dérivés indique que ces produits bactériens sont capables de stimuler l'immunité générale de l'organisme.

II apparaît que si la FHA se révèle comme particulièrement performante comme adjuvant pour une réponse spécifique (figure 8a), la FHA apparaît comme un meilleur immunostimulant des IgG non spécifiques.

### EXEMPLE 9 :

### La FHA induit localement une augmentation d'ARNm codant pour le complexe majeur d'histocompatibilité MHCII et pour la molécule de costimulation B7-1.

Des souris OF1 ont été administrées par voie nasale avec 5 µg de FHA ou 20 µg de LPS ou seulement de l'eau physiologique apyrogène. Après différentes périodes de temps (1 h, 2 h, 4 h, 8 h, 12 h, 24 h, 48 h) les souris ont été sacrifiées et les poumons prélevés. L'analyse par RT-PCR des ARNm de ces extraits a permis d'observer une augmentation des ARNm codant pour le complexe majeur d'histocompatibilité MHCII et pour la molécule de costimulation B7-1 dans le groupe FHA par rapport au groupe eau physiologique. En revanche le niveau d'expression des mRNA des différentes cytokines étudiées n'a présenté aucune différence entre ces deux groupes alors qu'une forte augmentation était observée dans le groupe LPS témoin.

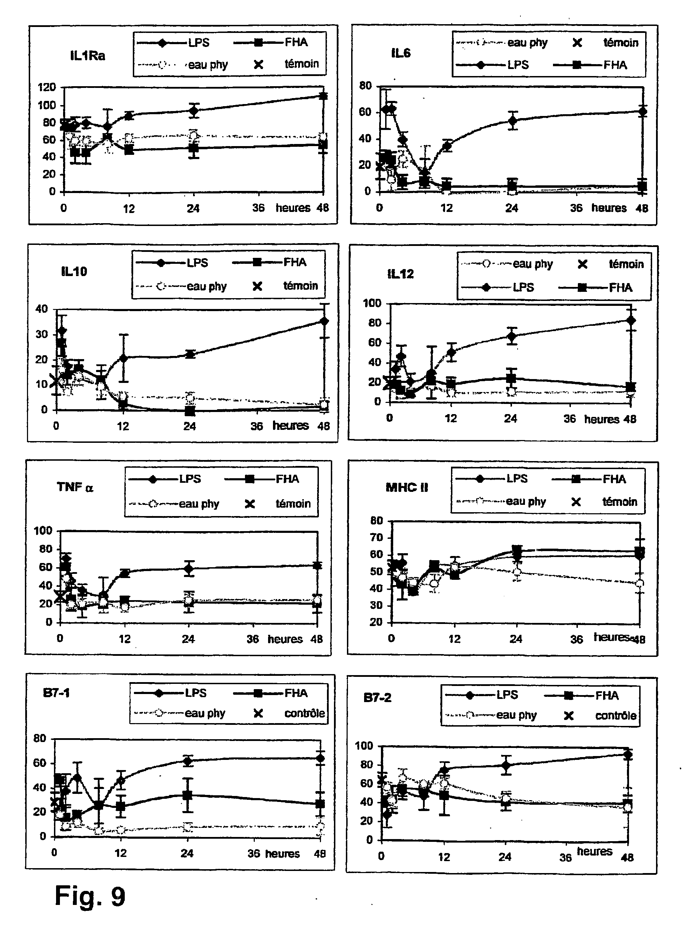

Les résultats obtenus sont illustrés dans la figure 9.

L'augmentation de MHCII et de B7-1 suggère une augmentation de la présentation au niveau local induite par la FHA qui pourrait en partie expliquer son activité adjuvante. De plus, on peut noter que l'absence d'une surexpression des cytokines pro-inflammatoires induite par la FHA est une qualité supplémentaire de cette molécule en vue de son utilisation comme adjuvant.

### REFERENCES BIBLIOGRAPHIQUES

(1) • LOCHT, C., BERTIN P., MENOZZI F.D., and RENAUD G. (1993). The filamentous haemagglutinin, a multifaced adhesin produced by virulent *Bordetella sp.* Mol. Microbiol. 9: 653-660.
(2) • BROWN, D.R., and PARKER, C.D. (1987). Cloning of the filamentous hemagglutinin of *Bordetella pertussis* and its expression in Escherichia coli. Infect. Immun., 55:154-161.
(3) • RELMAN D., DOMENIGHINI, M., TUOMANEN, E., RAPPUOLI, R. and FALKOW, S. (1989). Filamentous hemagglutinin of *Bordetella pertussis:* nucleotide sequence and crucial role in adherence. Proc. Natl. Acad. Sci. USA, 86:2637-2641.
(4) • DELISSE-GATHOYE, A. M., LOCHT, C., JACOB, F., RAASCHOU-NIELSEN, M., HERON, I., RUELLE, J.L., DE WILDE, M., and CABEZON, T. (1990). Cloning, partial sequence, expression and antigenic analysis of the filamentous hemagglutinin gene of *Bordetella pertussis.* Infect. Immun. 58:2895-2905.
(5) • MAKHOV, A.M., HANNAH, J.H., BRENNAN, M.J., TRUS, B.L., KOCSIS, E., CONWAY, J.F., WINGFIELD, P.T., SIMON, M.N., and STEVEN, A.C. (1994). Filamentous hemagglutinin of *Bordetella pertussis;* a bacterial adhesin formed as a 50nm monomeric rigid rod based on a 19-residue repeat motif rich in β-strands and turns. J. Mol. Biol., 241:110-124.
(6) • RENAULD-MONGENIE, G., CORNETTE, J., MIELCAREK, N., MENOZZI, F.D., and. LOCHT, C. (1996). Distinct roles of the N-terminal and C-terminal precursor domains in the biogenesis of the *Bordetella pertussis* filamentous hemagglutinin. J. Bacteriol. 178:1053-1060.
(7) • GREGORIADIS, G. (1990). Immunological adjuvants: a role for liposomes. Immunol. Today 11: 89-97.
(8) • ELDRIDGE, J. H., GILLEY, R. M., STAAS, J. K., MOLDOVEANU, Z., MEULBROEK, J. A., and TICE, T. R. (1989). Biodegradable microspheres: vaccine delivery system for oral immunization. Curr. Top. Microbiol. Immunol. 146: 59-66.
(9) • MOWAT, A. M. and DONACHIE, A. M. (1991). ISCOMs-a novel strategy for mucosal immunization? Immunol. Tod. 12: 383-385.
(10) • RENAULD-MONGENIE, G., MIELCAREK, N., CORNETTE, J., SCHACHT, A.M., CAPRON, A., RIVEAU, G., and LOCHT, C. (1996). Induction of mucosal immune responses against a heterologous antigen fused to filamentous hemagglutinin after intranasal immunization with recombinant *Bordetella pertussis.* Proc. Natl. Acad. Sci. USA 93: 7944-7949.
(11) • MIELCAREK, N., RIVEAU, G., REMOUE, F., ANTOINE, R., CAPRON, A., and LOCHT, C. (1998). Homologous and heterologous protection after single intranasal administration of live attenuated recombinant *Bordetella pertussis.* Nature Biotech. 16: 454-457.
(12) • POULAIN-GODEFROY, 0., MIELCAREK, N., IVANOFF, N., REMOUE, F., SCHACHT, A.M., PHILLIPS, N., LOCHT, C., CAPRON, A. and RIVEAU, G. (1998). Enhancement of immunogenicity by intranasal antigen delivery using liposomes bearing the *Bordetella pertussis* filamentous hemagglutinin. Infect. Immun. 66: 1764-1767.
(13) • HANNA, J. H., MENNOZI, F.D., RENAULD, G., LOCHT, C., and BRENNAN, M.J. (1994). Sulfated glycoconjugate receptors for the *Bordetella pertussis* adhesin filamentous hemagglutinin (FHA) and mapping of the heparin-binding domain on FHA. Infect. Immun, 62: 5010-5019.
(14) • KOCH Y., BARAM, T., HAZUM, E., and FRIDKIN, M. (1977). Resistance to enzymic degradation of LH-RH analogues possessing increased biological activity. Biochem. Biophys. Res. Commun., 74:488-491.

## Revendications

1. Utilisation de la protéine FHA ou d'une hémagglutinine filamenteuse ayant au moins 70% de similarité des séquences avec la FHA de *B. pertussis* et augmentant la réponse immunitaire spécifique d'un antigène, sous forme libre, pour la préparation des adjuvants.

2. Utilisation selon la revendication 1, dans laquelle l'hémagglutinine filamenteuse ayant au moins 70% de similarité des séquences avec la FHA de *B. pertussis* et augmentant la réponse immunitaire spécifique d'un antigène est obtenue par substitution conservative des acides aminés.

3. Utilisation de la protéine FHA selon l'une des revendications 1 ou 2 dans laquelle la protéine est rendue résistante à la protéolyse par remplacement de la liaison peptidique -CONH- par une liaison réduite -CH₂NH-, une liaison retro-inverso -NHCO-, une liaison méthylène-oxy CH₂O, une liaison carba - CH₂CH₂-, une liaison cétométhylène -CO-CH₂, une liaison hydroxyéthylene - CHOH-CH₂, une liaison -N-N, une liaison E-alcène ou encore une liaison CH=CH.

## Claims

1. Use of FHA protein or of a filamentous haemagglutinin having at least 70% sequence similarity with the FHA of *B. pertussis* and augmenting the specific immune response of an antigen, in the free form, for the preparation of adjuvants.

2. Use according to claim 1, in which the filamentous haemagglutinin having at least 70% sequence similarity with the FHA of *B. pertussis* and augmenting the specific immune response of an antigen is obtained by conservative amino acid substitution.

3. Use of FHA protein according to claim 1 or claim 2, in which the protein is rendered resistant to proteolysis by replacing the peptide linkage -CONH- by a reduced linkage -CH₂NH-, a retro-inverso linkage -NHCO-, a methylene-oxy linkage CH₂O, a carba linkage -CH₂CH₂-, a ketomethylene linkage -CO-CH₂, a hydroxyethylene linkage -CHOH-CH₂, a -N-N linkage, a E-alkene linkage or a CH=CH linkage.

## Patentansprüche

1. Verwendung des FHA-Proteins oder eines filamentösen Hämagglutinins mit mindestens 70 % Sequenzähnlichkeit mit dem FHA von *B. pertussis* und das, in freier Form, die spezifische Immunantwort eines Antigens verstärkt, für die Herstellung von Adjuvantien.

2. Verwendung nach Anspruch 1, bei der das filamentöse Hämagglutinin, das mindestens 70 % Sequenzähnlichkeit mit dem FHA von *B. pertussis* aufweist und die spezifische Immunantwort eines Antigens verstärkt, durch konservative Substitution von Aminosäuren erhalten ist.

3. Verwendung des FHA-Proteins nach einem der Ansprüche 1 oder 2, bei der das Protein Beständigkeit gegen Proteolyse erhalten hat durch Ersetzen der Peptidbindung -CONH- durch eine reduzierte Bindung -CH₂NH-, eine retroinverse Bindung -NHCO-, eine Methylenoxybindung -CH₂O-, eine Carbonbindung -CH₂CH₂-, eine Ketomethylenbindung -CO-CH₂, eine Hydroxyethylenbindung -CHOH-CH₂, eine Bindung -N-N, eine E-Alkenbindung oder eine Bindung CH=CH.
